Europäisches Patentamt

(19) European Patent Office

Office européen des brevets

(11) Publication number: **0 269 419**
**B1**

(12) **EUROPEAN PATENT SPECIFICATION**

(45) Date of publication of patent specification: **16.01.91**

(21) Application number: **87310365.9**

(22) Date of filing: **24.11.87**

(51) Int. Cl.⁵: **B 29 C 65/18, A 61 F 5/44**

(54) **Manufacture of bags.**

(30) Priority: **28.11.86 GB 8628480**
**28.11.86 GB 8628481**

(43) Date of publication of application:
**01.06.88 Bulletin 88/22**

(45) Publication of the grant of the patent:
**16.01.91 Bulletin 91/03**

(84) Designated Contracting States:
**AT BE CH DE ES FR GB IT LI LU NL SE**

(56) References cited:
**GB-A-1 376 768**
**GB-A-2 181 951**
**US-A-3 205 889**
**US-A-3 337 074**
**US-A-3 788 324**
**US-A-4 425 177**

(73) Proprietor: **E.R. Squibb & Sons, Inc.**
**Lawrenceville-Princeton Road**
**Princeton, N.J. 08540-4000 (US)**

(72) Inventor: **Steer, Peter Leslie**
**"Kingscote" Woodlands Rise**
**East Grinstead Sussex (GB)**
Inventor: **Steer, Graham Emery**
**Paygate Cottage Cotchford Hill**
**Hartfield East Susex (GB)**

(74) Representative: **Cook, Anthony John et al**
**D. YOUNG & CO. 10, Staple Inn**
**London, WC1V 7RD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to the manufacture of bags for containing liquids, and to the bags themselves.

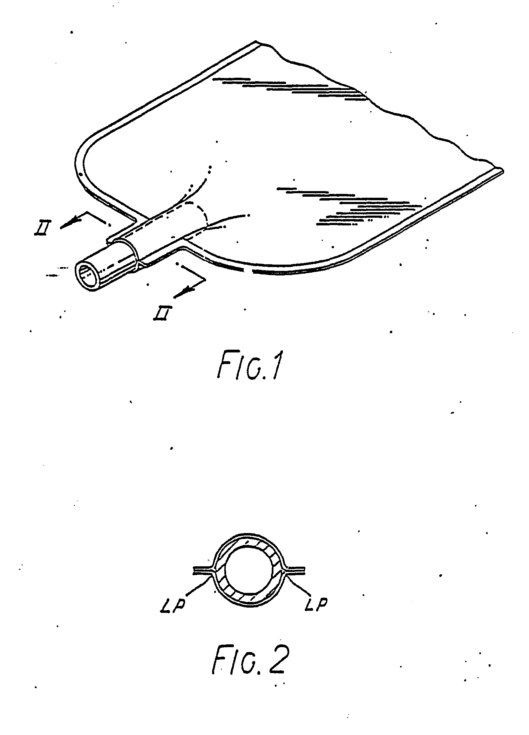

Bags such as urostomy bags, urine bags, colostomy bags, etc. are commonly made from two superposed sheets of plastic material welded around their periphery. It is often desired to have an outlet tube from a lower region of the bag. One example of a urine bag with two superposed walls and a tube is seen in British Patent Application No. 2 181 951. Such tubes are often also plastics material. Problems arise whem making weld joints to fix the tube to the two bag walls in a leak-proof manner. In particular, there frequently exist two leak paths LP at the locations indicated in Fig. 2 of the accompanying drawings. This problem is particularly acute with bags for containing urine because urine has a low surface tension and will readily find any leak path. Welding a plastics tube between bag walls is a particularly difficult problem when one is employing multi-film laminate material for each bag wall, some of the layers of the laminate being intended to provide strength and liquid impermeability and one or more layers of the laminate being particularly directed to providing gas impermeability. As will be understood, with a thin multi-laminate bag wall, joining such a wall to a tube of appreciable wall thickness presents difficulties in delivering a suitable amount of heat both to the thin bag wall film and to the relatively thick tube wall.

According to the present invention, there is provided a method for making a bag for containing liquids and having an outlet tube characterised by placing the tube between two superposed bag walls, lightly seam welding the walls to hold the tube in position, placing a two-part mold over the tube and the end of the bag and then admitting molten plastics material into the mold to form a liquid tight seal between the tube and the bag.

Also according to the invention, there is provided a method for manufacturing a bag for containing liquids comprising the step of seal or tack welding an outlet tube in position between superposed bag walls and, as a second step, crimping a sleeve around parts of the bag walls and the tube, the sleeve being crimped on with sufficient force to close off any leak paths at the junction of the tube and bag walls.

In this method, one can employ a crimpable plastic sleeve, or a crimpable metal sleeve, or a composite sleeve made of both metal and plastic. The basis of this invention is that the application of crimping force in the direction indicated will cause the bag wall strips on either side of the tube to be compressed under high pressure towards and into the adjacent portion of the tube wall, which reduces the possibility of the leak paths LP being present in the manufactured product.

In an embodiment of the invention as first mentioned above, a tube is held in position between two superimposed walls of a bag by a light seam welding operation, without taking any special precautions, at this point, to make the joint leakproof. Thereafter, the bag and tube combination is placed between the blocks of a mold, each having confronting recesses of a particular shape, and molten plastic is injected to fill these recesses, the injection being done, as normal, under heat and pressure. The injected material forms a collar which completely surrounds the tube, the bag film material on either side of the tube having been melted to itself and to the tube wall and the collar being securely attached in an encircling configuration to preclude leakage.

In the accompanying illustrative drawings:

Fig. 1 is a perspective view of a bag having an outlet tube attached thereto in a conventional manner;

Fig. 2 is a cross-section on the line II—II of Fig. 1 illustrating the two leak paths LP which frequently occur with this (prior art) method despite all efforts to avoid them;

Fig. 3 illustrates the first step of one example of method according to the present invention, in perspective view;

Fig. 4 illustrates a later step of method in which a sleeve is placed in position for crimping;

Fig. 5 is a perspective view showing a crimped-on sleeve;

Fig. 6 is a cross-sectional view on the sectional plane VI—VI illustrating the completed bag having a crimped-on sleeve;

Fig. 7 is a perspective view of a bag with a tube inserted and a mold over the tube in the manner of an alternative embodiment of the invention;

Fig. 8 is a perspective view of a portion of the mold used in conjunction with the invention as illustrated in Fig. 7; and

Fig. 9 is a perspective view of the completed bag made in accordance with the alternative embodiment of the invention.

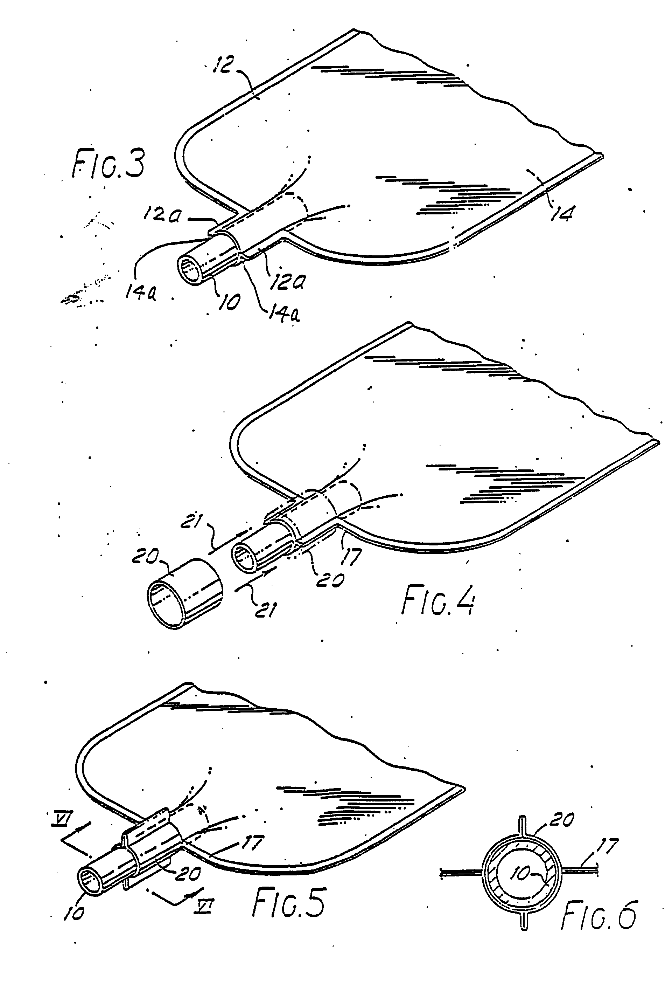

Referring to Figs. 3—6, in one method according to the invention, the tube 10 is lightly seam welded by the illustrated seam weld between the two superposed bag films 12 and 14. This weld join may be quickly made as it is not necessary that the joint be leak-proof. Its purpose is to hold the parts in position during the next step. A crimpable sleeve 20, e.g., of metal or plastic material, is then placed over the tube and pushed towards the bag (as indicated by arrows 21) so that the sleeve surrounds the parts 12a, 14a, of the superposed bag walls and one end of the sleeve lightly abuts the edge 17. Using a conventional crimping tool, and applying the crimping force in a direction substantially at 90° to the plane of the bag walls, the sleeve is next crimped tightly onto and over the tube and the wall portions 12a, 14a. Because the force is applied in the indicated direction, the material of the sleeve is forced under high pressure towards those regions of the bag wall material adjacent to the leak paths LP and the sleeve when crimped forces this material into tight engagement with the exterior surface of the tube. At the end of crimping, the sleeve has taken up a permanent set and tightly holds the bag wall material now squashed out of strip form to close off the leak paths LP.

One advantage of the method particularly disclosed herein is that the crimping being a fast operation, high productivity can be obtained without compromising quality.

In the present specification, a reference has been made to applying the crimping force at right angles to the plane of the walls of the bag. The purpose of this is to be sure of compressing the bag wall strips tightly against the tube. Angles other than 90° could be used, e.g., an angle in the range of 75—90° would be suitable in many cases, and the invention is considered to include this possblity.

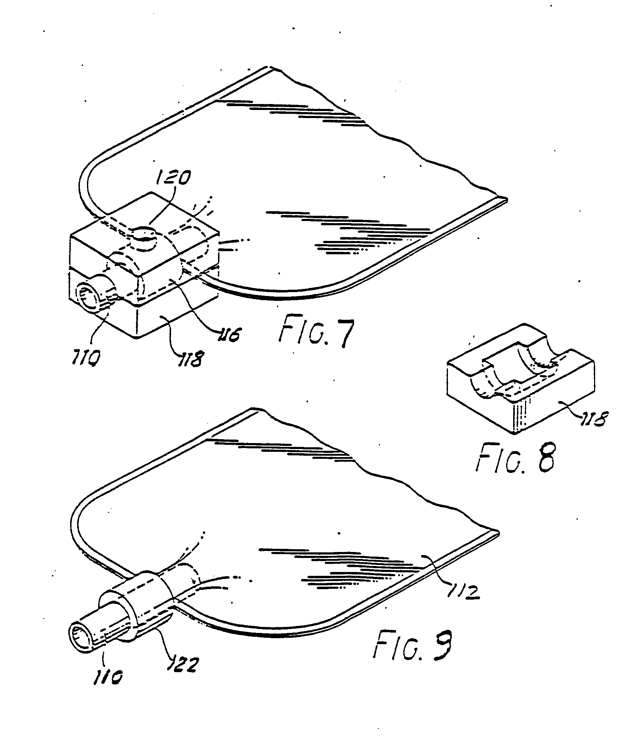

With reference to Figs. 7—9, an alternative embodiment of the present invention is illustrated. In the alternative embodiment, a tube 110 is inserted into an opening at one end of the bag. No special precautions need be taken with respect to sealing the tube within the bag opening.

A mold having upper and lower portions 116, 118 is applied over the tube 110 where it enters the bag, and molten plastic is admitted into the mold through an opening 120 in the upper portion 116. When the plastic has cured, it forms a fluid tight collar 122 as it will bond both to the bag and to the tube.

## Claims

1. A method for making a bag for containing liquids and having an outlet tube characterised by placing the tube between two superposed bag walls, lightly seam welding the walls (112) to hold the tube (110) in position, placing a two-part mold (116, 118) over the tube (110) and the end of the bag and then admitting molten plastics material into the mold to form a liquid tight seal between the tube and the bag.

2. A method for manufacturing a bag for containing liquids comprising the step of seal or tack welding an outlet tube in position between superposed bag walls and, as a second step, crimping a sleeve around parts of the bag walls and the tube, the sleeve being crimped on with sufficient force to close off any leak paths at the junction of the tube and bag walls.

3. A method according to claim 2 in which the crimping force is applied at an angle of 75—105° to the plane of the bag walls.

4. A method according to claim 3 in which the said angle is substantially 90°.

## Patentansprüche

1. Verfahren zum Herstellen eines Beutels zur Aufnahme von Flüssigkeiten mit einem Auslaßrohr, gekennzeichnet durch Anordnen des Rohrs zwischen zwei übereinandergelegten Beutelwänden, leichtes Nahtschweißen der Wände (112), so daß das Rohr (110) in Position gehalten wird, Anordnen einer zweiteiligen Form (116, 118) über das Rohr (110) um das Ende des Beutels und dann Einlassen von geschmolzenem Kunststoffmaterial in die Form, so daß eine flüssigkeitsdichte Abdichtung zwischen dem Rohr und dem Beutel gebildet wird.

2. Verfahren zum Herstellen eines Beutels zur Aufnahme von Flüssigkeiten mit dem Schritt Naht- oder Heftschweißen eines Auslaßrohrs in Position zwischen übereinandergelegten Beutelwänden und als ein zweiter Schritt, Aufquetschen einer Manschette um Teile der Beutelwände und des Rohrs, wobei die Manschette mit ausreichender Kraft aufgequetscht wird, so daß jegliche Leckstellen an der Verbindung des Rohrs und den Beutelwänden verschlossen werden.

3. Verfahren nach Anspruch 2, wobei die Quetschkraft in einem Winkel von 75 bis 105° zur Ebene der Beutelwände aufgebracht wird.

4. Verfahren nach Anspruch 3, wobei der genannte Winkel im wesentlichen 90° beträgt.

## Revendications

1. Procédé de fabrication d'un sac destiné à contenir des liquides et comportant un tube de sortie, caractérisé en ce qu'on place le tube entre deux parois de sac superposées, on procède à un léger soudage continu des parois (112) pour maintenir le tube (110) en position, on place un moule en deux parties (116, 118) par dessus le tube (110) et l'extrémité du sac, puis on fait arriver de la matière plastique fondue dans le moule pour former un joint étanche aux liquides entre le tube et le sac.

2. Procédé de fabrication d'un sac destiné à contenir des liquides, comprenant le soudage d'étanchéité ou de pointage d'un tube de sortie en position entre des parois de sac superposées, et dans un second temps, le sertissage d'un manchon autour de parties des parois du sac et du tube, le manchon étant serti avec une force suffisante pour obturer tous chemins de fuite à la jonction du tube et des parois du sac.

3. Procédé selon la revendication 2, dans lequel on applique la force de sertissage sous un angle de 75—105° par rapport au plan des parois du sac.

4. Procédé selon la revendication 3, dans lequel ledit angle est sensiblement égal à 90°.

Fig.1

Fig. 2

LP          LP

FIG.3

FIG.4

FIG.5

FIG.6

FIG. 7

FIG. 8

FIG. 9